# EUROPÄISCHE PATENTSCHRIFT

(11) **EP 1 071 395 B1**
(45) Veröffentlichungstag und Bekanntmachung des Hinweises auf die Patenterteilung: **20.11.2002**
(21) Anmeldenummer: 99919196.8
(22) Anmeldetag: 06.04.1999
(51) Int. Cl.: A61K 7/48

(54) **MILD ABRASIVE HAUTREINIGUNGSMITTEL**
MILDLY ABRASIVE SKIN CLEANING AGENTS
AGENTS NETTOYANTS POUR LA PEAU, PEU ABRASIFS

(30) Priorität: 15.04.1998 DE 19816664
(43) Veröffentlichungstag der Anmeldung: 31.01.2001
(73) Patentinhaber: Henkel Kommanditgesellschaft auf Aktien, 40589 Düsseldorf (DE)
(72) Erfinder: SCHELGES, Heike, D-47807 Krefeld (DE); SCHOSSER, Gryta, D-47829 Krefeld (DE)
(86) Internationale Anmeldenummer: EP9902321
(87) Internationale Veröffentlichungsnummer: WO99052500

(56) Entgegenhaltungen:
- EP-A- 0 295 886
- WO-A-95/11005
- WO-A-97/31619
- US-A- 5 534 265
- US-A- 5 578 312
- US-A- 5 665 687

## Beschreibung

Zur Reinigung der Haut werden üblicherweise wäßrige Tensidlösungen verwendet. Damit lassen sich aber festsitzende Verunreinigungen, abgestorbene Hautschuppen, Talgabsonderungen und Schminke nur unvollständig entfernen.

Es wird daher empfohlen, die Haut in regelmäßigen Zeitabständen einer Tiefenreinigung zu unterziehen, bei der zusätzliche mechanische Hilfsmittel, z.B. ein Schwämmchen, ein Reinigungstuch oder eine abrasive bzw. exfoliative Reinigungszusammensetzung verwendet wird. Die Verwendung mild abrasiver Hautreinigungsmittel ist auch zur Behandlung von Akne und anderen Hautkrankheiten vorgeschlagen worden, um die Regeneration des Hautgewebes zu fördern und die Penetration von danach aufgebrachten dermatologischen Präparaten zu begünstigen.

Während man zur Reinigung verschmutzter Hände Handwaschpasten mit relativ groben Abrasiva wie z.B. Bimssteinmehl, Seesand, Sägemehl oder Kreide einsetzt, werden für die Tiefenreinigung empfindlicher Haut, z.B. des Gesichts, weichere oder feiner gemahlene Abrasiva verwendet, z.B. feinteilige Polymerpulver, feinteilige Wachs- oder Triglycerid-Pulver und vor allem pflanzliche Abrasivstoffe. Aus EP 0 571 193 A1 waren z.B. abrasive Zusammensetzungen mit einem Gehalt an mineralischen Abrasivkomponenten bekannt.

Der Wunsch nach kosmetischen Reinigungsmitteln auf natürlicher Basis hat vor allem die Verwendung pflanzlicher Abrasiva gefördert. Außer den Tensiden und Abrasivstoffen enthalten die wäßrigen Hautreinigungsmittel üblicherweise ein Quellmittel oder ein wasserlösliches Verdickungsmittel, da mit dem Abrasivum allein die Einstellung definierter Viskositäten schwer oder gar nicht möglich ist. Es hat sich nun gezeigt, daß in Gegenwart pflanzlicher Abrasiva die meisten Quellmittel oder wasserlöslichen Hydrocolloide auf Basis natürlicher Rohstoffe, z.B. Quellstärken, Cellulosederivate und Guar und deren wasserlöslichen Derivate oder viele Pflanzengumme durch enzymatische Vorgänge abgebaut werden und bei längerer Lagerung ihre Viskosität verlieren und zu Stabilitätsproblemen (ggf. Produkttrennung) führen können.

Es wurde daher nach Möglichkeiten gesucht, milde abrasive Hautreinigungsmittel mit einem Gehalt an pflanzlichen Abrasivstoffen stabil und dauerhaft zu verdicken. Es wurde dabei überraschend festgestellt, daß Biopolymere aus der Gruppe der extracellulären mikrobiellen Polysaccharide gegenüber den durch pflanzliche Abrasiva eingetragenen saccharidspaltenden Enzymen stabil sind.

Gegenstand der Erfindung sind daher wäßrige Hautreinigungspräparate in Form von viskosen Lotionen, Cremes oder Gelen mit einem Gehalt an pflanzlichen Abrasivstoffen und wasserlöslichen Verdickungsmitteln, dadurch gekennzeichnet, daß als wasserlösliche Verdickungsmittel Biopolymere aus der Gruppe der extracellulären mikrobiellen Polysaccharide enthalten sind.

Als mikrobielle Polysaccharide mit verdickenden Eigenschaften auf wäßrige Zubereitungen sind vor allem Xanthan-Gum (von Xanthomones campestris), Gellan (von Pseudomonas elodia), Welan, Rhamsan und Curdlan (von Alcaligenes spp.), Pullulan (aus Aureobasidium pullulans), Scleroglucan und andere extracelluläre Biopolymere geeignet. Bevorzugt, insbesondere wegen seiner technischen Verfügbarkeit, ist Xanthan-Gum, der u.a. unter den Handelsbezeichnungen Keltrol®, Kelzan®, Actigum®, Biozan®, Rhodigel®, Rhodopol®, Satiaxane® und Shellflo® von verschiedenen Herstellern angeboten wird.

Die erfindungsgemäßen milden abrasiven Hautreinigungsmittel enthalten als pflanzliche Abrasiva z.B. das gemahlene Endocarp von Aprikosen-, Pfirsich- oder Walnuß- oder Kirsch-Kernen oder das gemahlene, ggf. entfettete Fruchtfleisch von Mandeln, Kokosnüssen, Jojobafrüchten, Macadamia-Nüssen und anderen Nüssen. Weitere als milde Abrasiva geschätzte Pflanzenmehle sind z.B. Maiskolbenmehl, Weizenkleie, Hafermehl und Holzmehl.

Die als milde Abrasiva geeigneten pflanzlichen Rohstoffe weisen bevorzugt eine Teilchengröße von 50 - 1000 µm, bevorzugt von 100 - 500 µm, auf, die durch Mahlung und Klassifizierung durch Siebe eingestellt werden kann. Solche Abrasiva werden üblicherweise in Mengen von 0,5 - 70 Gew.-%, bezogen auf das gesamte Hautreinigungsmittel, verwendet. Für die Zwecke der vorliegenden Erfindung eignen sich als Abrasivstoffe pflanzlichen Ursprungs ganz besonders Naturmehle aus gemahlenen Kernen, Fruchthülsen oder Samen in einer Menge von 0,5 - 30 Gew.-% der Reinigungsmittel.

Die wasserlöslichen Verdickungsmittel aus der Gruppe der extracellulären mikrobiellen Polysaccharide werden üblicherweise in Mengen von 0,05 - 10 Gew.-% eingesetzt. Ganz besonders für die Zwecke der vorliegenden Erfindung eignet sich als wasserlösliches Verdickungsmittel der Xanthan-Gum in einer Menge von 0,1 - 5 Gew.-% des Hautreinigungsmittels.

Zusätzlich zu den pflanzlichen Abrasivstoffen und den wasserlöslichen Verdickungsmitteln können auch andere z.B. anorganische und organische Abrasivkomponenten und anorganische oder synthetische organische Verdickungsmittel enthalten sein. Als anorganische Abrasiv-komponente sind z.B. Kreide (Calcit), Aluminiumoxid, Aluminiumoxidtrihydrat, Calciumphosphat-dihydrat (CaHPO₄·2H₂O), Calciumpyrophosphat, unlösliches Natrium-metaphosphat und Kieselsäuren (Fällungs- und Gel-Kieselsäuren) geeignet. Als organische Abrasivstoffe eignen sich z.B. Polyethylen- oder Polyamid-Pulver.

Als anorganische Verdickungsmittel eignen sich z.B. Schichtsilikate, z.B. Bentonit, Montmorillonit, Kaolin. Talkum und organisch modifizierte Schichtsilikate, Alumosilikate, Veegum® und pyrogene Kieselsäuren. Als synthetische organische Verdickungsmittel können zusätzlich z.B. wasserlösliche Polyethylenoxide, Polyurethane, Polyvinylpyrrolidon, Polyvinylalkohol und Polyacrylamide eingesetzt werden.

Weiterhin können die erfindungsgemäßen abrasiven Hautreinigungsmittel oberflächenaktive Stoffe enthalten. Geeignet sind dafür anionische, zwitterionische, ampholytische und nichtionische oberflächenaktive Stoffe oder Gemische davon. Diese sind bevorzugt in Mengen von 0,1 - 30 Gew.-% in den Hautreinigungsmitteln enthalten. Alle geeigneten Tenside zeichnen sich durch eine bevorzugt lineare Alkylgruppe oder Acylgruppe mit 8 - 22 C-Atomen und eine polare, ionische oder nichtionische Gruppierung aus. Beispiele für besonders geeignete oberflächenaktive Stoffe sind vor allem die gut wasserlöslichen und in wäßrigem Medium Schaum erzeugenden anionischen Tenside wie z.B die Alkylsulfate und Alkylethersulfate, die Alkyl-(polyglycolether)-carboxylate, die Sulfobernsteinsäurealkylester-Salze, Alkansulfonate, α-Olefinsulfonate, Acylsarkosinate, Acyltauride und Acylisethionate.

In Kombination mit anionischen Tensiden können auch zwitterionische und ampholytische Tenside oder nichtionische Tenside eingesetzt werden. Geeignete zwitterionische Tenside sind z.B. die Alkyl-dimethyl-carboxymethylammonium-Tenside (Betain-Tenside), die Acylamidoethyl-dimethyl-acetobetaine, die Imidazoliniumbetaine und Sulfobetaine. Geeignete Ampholyte sind z.B. N-Alkylaminocarbonsäuren, die durch Anlagerung von Alkylaminen an Acrylsäure oder Crotonsäure erhältlich sind.

Als nichtionische Tenside sind zunächst die Anlagerungsprodukte von Ethylenoxid an Fettalkohole, an Fettsäuren, an Fettsäurepolyol-partialester, z.B. an Monoglyceride, Sorbitanfettsäureester oder an Methylglucosid-fettsäureester zu nennen. Bevorzugte nichtionogene Tenside sind aber vor allem die Alkylglucoside, die aus Glucose durch Acetalisierung mit Fettalkoholen zugänglich sind.

Dabei werden Oligoglucoside erhalten, die einen Mono- oder Oligoglucosidrest aus 1 - 4 Glucoseeinheiten enthalten. Besonders geeignete Alkylglucoside weisen einen mittleren Oligomerisationsgrad von 1 - 2, bevorzugt von 1,5 auf.

Die erfindungsgemäßen abrasiven Hautreinigungsmittel können als fließfähige Lotion mit Viskositäten von 1 - 500 Pa·s (20° C) oder als Pasten mit Viskositäten von mehr als 500 Pa·s (20° C) vorliegen. Sie können zur Verdickung und zur Verbesserung der hautkosmetischen Eigenschaften auch emulgierte Öl- oder Fettkomponenten oder dispergierte Wachse oder andere schmelzbare Fettkomponenten enthalten. Solche dispergierten Fettstoffteilchen verbessern auch gleichzeitig die mild abrasiven Eigenschaften und werden gelegentlich als Abrasivkomponenten bezeichnet.

Weitere fakultative Komponenten der erfindungsgemäßen Hautreinigungsmittel sind Konservierungsstoffe, pH-Stellmittel bzw. Puffersalze, Farbstoffe, Duftstoffe, Konservierungsstoffe, wasserlösliche Glycole oder Polyole mit 2 - 10 C-Atomen, wie z.B. Glycerin, Sorbit oder Propylenglycol, in einer Menge von 1 - 20 Gew.-%, Polyethylenglycole in einer Menge von 1 - 20 Gew.-%, sowie ggf. kosmetische oder dermatologische Wirkstoffe, z.B. antimikrobielle, sebosuppressive, antiphlogistische (entzündungshemmende) Wirkstoffe, Pflanzenextrakte, Vitamine und Vitaminoide, Proteine und Proteinderivate.

Ein weiterer Gegenstand der Erfindung sind Hautreinigungsmittel mit einem Gehalt von 0,5 - 30 Gew.-% eines pflanzlichen Abrasivstoffs, gegebenenfalls 0 - 20 Gew.-% anderer Abrasivstoffe, 0,1 - 5 Gew.-% eines mikrobiellen Polysaccharid-Verdickungsmittels, 0,1 - 30 Gew.-% anionischer, zwitterionischer, amphoterer oder nichtionischer Tenside, 1 - 20 Gew.-% wasserlöslicher Glycole oder Polyole mit 2-10 C-Atomen oder Polyethylenglycole und 20 - 40 Gew.-% Wasser.

Die folgenden Beispiele sollen den Gegenstand der Erfindung näher erläutern, ohne ihn hierauf zu beschränken :

### Beispiele

Es wurden die folgenden Rezepturen für Hautpeeling-Präparate hergestellt:

| | **1** | **1V** |
|---|---|---|
| Texapon N70 (1) | 9 | 9 |
| Dehyton G (3) | 3 | 3 |
| Emulgade 1000 NI (5) | 4 | 4 |
| 1,2-Propylenglycol | 4 | 4 |
| Sorbit (70 %ig) | 10 | 10 |
| Parfümöl | 0,1 | 0,1 |
| Konservierungsmittel (6) | 1,2 | 1,2 |
| Xanthan-Gum (7) | 0,5 | - |
| Carboxymethylcellulose | - | 1,0 |
| Mandelmehl, süß | 12 | 12 |
| Maisstärke | 3,6 | 3,6 |
| Kaolin | 3,6 | 3,6 |
| Citronensäure bis pH = 5 ± 0,5 | 0,9 | 0,9 |
| Wasser | ad 100 | ad 100 |
| Viskosität (Pa·s) 20°C nach Herstellung | 400 | 437 |
| nach 24 Stunden | 443 | 362 |
| nach 48 Stunden | 500 | 312 |
| nach 6 Tagen | 575 | 225 |

Es wurden die folgenden erfindungsgemäßen Hautpeeling-Präparate hergestellt:

Die Herstellung erfolgte wie folgt:

Das Parfümöl wurde mit den Tensiden (Plantacare 1200, Dehyton G, Texapon NSO) gemischt, dann wurde der Xanthan-Gum darin aufgequollen und gelöst, das Konservierungsmittel (p-Hydroxybenzoesäureester, Na-Benzoat) zugesetzt und zuletzt das Naturmehl-Abrasivum (Mandelmehl, Jojobamehl, Maiskolbenmehl) unter Rühren darin dispergiert.

| | **2** | **3** | **4** | **5** |
|---|---|---|---|---|
| Texapon NSO (2) | 20 | - | - | 15 |
| Plantacare 1200 (4) | 10 | 15 | - | 10 |
| Dehyton G (3) | - | - | 4 | 3 |
| Parfümöl | 0,5 | 0,15 | 0,1 | - |
| p-Hydroxybenzoesäuremethylester | 0,2 | 0,2 | 0,2 | 0,2 |
| Na-Benzoat | 0,2 | 0,2 | 0,2 | 0,2 |
| Xanthan-Gum (7) | 0,5 | 1,0 | 1,5 | 2,0 |
| Mandelmehl | 15,0 | - | - | 15,0 |
| Jojobamehl | - | - | 5 | 5 |
| Maiskolbenmehl | - | 5 | - | 5 |
| Citronensäure bis pH = 5 ± 0,5 | 0,5 | 0,5 | 0,4 | 0,5 |
| Wasser | ad 100 | ad 100 | ad 100 | ad 100 |
| Viskosität (Pa·s) 20° C | | | | |
| nach Herstellung | 47,5 | 12,5 | 27,5 | 987,5 |
| nach 30 Tagen | 47,5 | 18,75 | 31,25 | 1125,0 |

Die Messung der Viskosität erfolgte mit einem Rotationsviskosimeter der Fa. Brookfield, Type RVF, Spindel TC (bzw. TE in Beispiel 1, 1V und 5) 4 RPM.

Folgende Rohstoffe bzw. Handelsprodukte wurden verwendet:

| | |
|---|---|
| (1) Texapon N70 | Na-Alkyl-(C₁₂/C₁₄)-polyglycolether(2 EO)sulfat, 70 %ige Lösung |
| (2) Texapon NSO | Na-Alkyl-(C₁₂/C₁₄)-polyglycolether(2 EO)sulfat, 28 %ige Lösung |
| (3) Dehyton G | N-(2-Hydroxyethyl)-N-(Kokosamidoethyl)-carboxymethyl-glycinat, Na-Salz, 30 %ige Lösung |
| (4) Plantacare 1200 | C₁₂-C₁₆-Alkyl-1,4-glucosid |
| (5) Emulgade 1000 NI | Gemisch aus Cetyl-/Stearylalkohol und Cetyl-/ Stearylalkohol-polyglycolether (20 EO) |
| (6) Konservierungsmittel | Gemisch aus PHB-Methylester, PHB-Propylester und Na-Benzoat (1 : 2 : 2) |
| (7) Xanthan-Gum | Keltrol® F |

## Patentansprüche

1. Wäßrige Hautreinigungsmittel in Form von viskosen Lotionen, Cremes oder Gelen, enthaltend
- pflanzliche Abrasivstoffe,
- Biopolymere aus der Gruppe der extracellulären mikrobiellen Polysaccharide als wasserlösliche Verdickungsmittel sowie
- anionische, zwitterionische oder ampholytische oberflächenaktive Stoffe.

2. Hautreinigungsmittel gemäß Anspruch 1, **dadurch gekennzeichnet, daß** als pflanzliche Abrasivstoffe Naturmehle aus gemahlenen Kernen, Schalen oder Samen, das gemahlene Endocarp von Aprikosen-, Pfirsich-, Walnuß- oder Kirschkernen, das gemahlene, gegebenenfalls entfettete Fruchtfleisch von Mandeln, Kokosnüssen, Jojobafrüchten, Macadamia-Nüssen und anderen Nüssen, Maiskolbenmehl, Weizenkleie, Hafermehl oder Holzmehl in einer Menge von 0,5 - 30 Gew.-% des gesamten Hautreinigungsmittels enthalten sind.

3. Hautreinigungsmittel nach einem der Ansprüche 1 oder 2, **dadurch gekennzeichnet, daß** als wasserlösliches Verdickungsmittel Xanthan-Gum in einer Menge von 0,1 - 5 Gew.-% des Hautreinigungsmittels enthalten ist

4. Hautreinigungsmittel nach einem der Ansprüche 1 - 3, **dadurch gekennzeichnet, daß** nichtionische oberflächenaktive Stoffe enthalten sind.

5. Hautreinigungsmittel nach einem der Ansprüche 1 - 4, **dadurch gekennzeichnet, daß** die oberflächenaktiven Stoffe in einer Menge von 0,1 - 30 Gew.-% enthalten sind.

6. Hautreinigungsmittel nach einem der Ansprüche 1 - 5, **dadurch gekennzeichnet, daß** wasserlösliche Glycole oder Polyole mit 2 - 10 C-Atomen oder Polyethylenglycole in einer Menge von 1 - 20 Gew.-% enthalten sind.

7. Hautreinigungsmittel, **gekennzeichnet durch** einen Gehalt von
0,5 - 30 Gew.-% eines pflanzlichen Abrasivstoffs, gegebenenfalls
0 - 20 Gew.-% anderer Abrasivstoffe,
0,1 - 5 Gew.-% eines mikrobiellen Polysaccharid-Verdickungsmittels,
0,1 - 30 Gew.-% anionischer, zwitterionischer, amphoterer oder nichtionischer Tenside,
1 - 20 Gew.-% wasserlöslicher Glycole oder Polyole mit 2 - 10 C-Atomen oder Polyethylenglycole,
20 - 40 Gew.-% Wasser.

8. Verwendung von Biopolymeren aus der Gruppe der extracellulären mikrobiellen Polysaccharide als gegen saccharidspaltende Enzyme stabiles wasserlösliches Verdickungsmittel wäßriger Hautreinigungsmittel, die pflanzliche Abrasivstoffe enthalten.

9. Verwendung gemäß Anspruch 8, **dadurch gekennzeichnet, daß** als pflanzliche Abrasivstoffe Naturmehle aus gemahlenen Kernen, Schalen oder Samen, das gemahlene Endocarp von Aprikosen-, Pfirsich-, Walnuß- oder Kirschkernen, das gemahlene, gegebenenfalls entfettete Fruchtfleisch von Mandeln, Kokosnüssen, Jojobafrüchten, Macadamia-Nüssen und anderen Nüssen, Maiskolbenmehl, Weizenkleie, Hafermehl oder Holzmehl in einer Menge von 0,5 - 30 Gew.-% des gesamten Hautreinigungsmittels enthalten sind.

10. Verwendung gemäß Anspruch 8 oder 9, **dadurch gekennzeichnet, daß** als wasserlösliches Verdickungsmittel Xanthan-Gum verwendet wird.

11. Verwendung gemäß einem der Ansprüche 8 - 10, **dadurch gekennzeichnet, daß** das Hautreinigungsmittel anionische, zwitterionische oder ampholytische oberflächenaktive Stoffe enthält.

12. Verwendung gemäß Anspruch 11, **dadurch gekennzeichnet, daß** das Hautreinigungsmittel nichtionische oberflächenaktive Stoffe enthält.

## Claims

1. A water-based skin cleansing preparation in the form of viscous lotions, creams or gels containing
- vegetable abrasives,
- biopolymers from the group of extracellular microbial polysaccharides as water-soluble thickeners and
- anionic, zwitterionic or ampholytic surfactants.

2. A skin cleansing preparation as claimed in claim 1, **characterized in that** natural meals of ground kernels, shells or seeds, the ground endocarp of apricot, peach, walnut or cherry kernels, the ground, optionally defatted fruit flesh of almonds, coconuts, jojoba fruits, macadamia nuts and other nuts, corn meal, wheat bran, oatmeal or wood meal are present as the vegetable abrasives in a quantity of 0.5 to 30% by weight, based on the skin cleansing preparation as a whole.

3. A skin cleansing preparation as claimed in claim 1 or 2, **characterized in that** xanthan gum is present as the water-soluble thickener in a quantity of 0.1 to 5% by weight.

4. A skin cleansing preparation as claimed in any of claims 1 to 3, **characterized in that** nonionic surfactants are present.

5. A skin cleansing preparation as claimed in any of claims 1 to 4, **characterized in that** the surfactants are present in a quantity of 0.1 to 30% by weight.

6. A skin cleansing preparation as claimed in any of claims 1 to 5, **characterized in that** water-soluble glycols or polyols containing 2 to 10 carbon atoms or polyethylene glycols are present in a quantity of 1 to 20% by weight.

7. A skin cleansing preparation, **characterized by** a content of
0.5 to 30% by weight of a vegetable abrasive, optionally
0 to 20% by weight of other abrasives,
0.1 to 5% by weight of a microbial polysaccharide thickener,
0.1 to 30% by weight of anionic, zwitterionic, amphoteric or nonionic surfactants,
1 to 20% by weight water-soluble glycols or polyols containing 2 to 10 carbon atoms or polyethylene glycols,
20 to 40% by weight water.

8. The use of biopolymers from the group of extracellular microbial polysaccharides as a water-soluble thickener resistant to saccharide-splitting enzymes in water-based skin cleansing preparations containing vegetable abrasives.

9. The use claimed in claim 8, **characterized in that** natural meals of ground kernels, shells or seeds, the ground endocarp of apricot, peach, walnut or cherry kernels, the ground, optionally defatted fruit flesh of almonds, coconuts, jojoba fruits, macadamia nuts and other nuts, corn meal, wheat bran, oatmeal or wood meal are present as the vegetable abrasives in a quantity of 0.5 to 30% by weight, based on the skin cleansing preparation as a whole.

10. The use claimed in claim 8 or 9, **characterized in that** xanthan gum is used as the water-soluble thickener.

11. The use claimed in any of claims 8 to 10, **characterized in that** the skin cleansing preparation contains anionic, zwitterionic or ampholytic surfactants.

12. The use claimed in claim 11, **characterized in that** the skin cleansing preparation contains nonionic surfactants.

## Revendications

1. Agent nettoyant pour la peau aqueux sous forme de lotions visqueuses, de crèmes ou de gels,
contenant :
- des substances abrasives végétales,
- des biopolymères choisis dans le groupe des polysaccharides microbiens extra cellulaires en tant qu'agent épaississant soluble dans l'eau ainsi que,
- des substances actives sur la tension superficielle anioniques, zwitterioniques ou ampholytiques.

2. Agent nettoyant pour la peau conformément à la revendication 1,
**caractérisé en ce que**
comme substance abrasive végétale des farines naturelles choisies parmi les noyaux moulés, les coques ou les graines, l'endocarpe moulu de noyaux d'abricot, de pèche, de noix ou de cerise, la chair de fuit moulue, éventuellement dégraissée d'amandes, de noix de coco, de fruits de jojoba, de noix de macadamia et d'autres noix, de la farine d'épi de maïs, du son de blé, de la farine d'avoine et de la farine de bois, sont contenues en une quantité allant de 0,5 à 30 % en poids, de l'agent nettoyant total.

3. Agent nettoyant pour la peau selon l'une quelconque des revendications 1 ou 2,
**caractérisé en ce que**
comme agent épaississant soluble dans l'eau, de la gomme xanthane est contenue en une quantité de 0,1 à 5 % en poids de l'agent nettoyant pour la peau.

4. Agent nettoyant pour la peau selon l'une quelconque des revendications 1 à 3,
**caractérisé en ce que**
des substances actives sur la tension superficielle non ioniques sont contenues.

5. Agent nettoyant pour la peau selon l'une quelconque des revendications 1 à 3,
**caractérisé en ce que**
les substances actives sur la tension superficielle sont contenues en une quantité de 0,1 à 30 % en poids.

6. Agent nettoyant pour la peau selon l'une quelconque des revendications 1 à 5,
**caractérisé en ce que**
des glycols solubles dans l'eau ou des polyols ayant de 2 à 10 atomes de carbone ou des polyéthylèneglycols sont contenus en une quantité de 1 à 20 % en poids.

7. Agent nettoyant pour la peau **caractérisé par** une teneur de :
0,5 à 30 % en poids d'une substance abrasive végétale, le cas échéant,
0 à 20 % en poids d'autres substances abrasives,
0,1 à 5 % en poids d'un agent épaississant du type polysaccharide microbien
0,1 à 30 % en poids d'agent tensioactif anionique, zwitterionique, amphotère ou non ionique,
1 à 20 % en poids de glycols solubles dans l'eau ou de polyols ayant de 2 à 10 atomes de carbone ou des polyéthylèneglycols,
20 à 40 % en poids d'eau

8. Utilisation de biopolymères choisis dans le groupe des polysaccharides microbiens extra cellulaires comme agent épaississant soluble dans l'eau, stable contre les enzymes qui clivent les saccharides d'agent aqueux nettoyant la peau qui contiennent des substances abrasives végétales.

9. Utilisation conformément à la revendication 8,
**caractérisée en ce que**
comme substances abrasives végétales des farines naturelles provenant de noyaux moulus, de coques ou de graines, l'endocarpe moulu de noyaux d'abricots, de pêches, de noix ou de cerises, la chair de fruit broyée, éventuellement dégraissée d'amandes, de farine d'épi de maïs, de son de blé, de farine d'avoine, ou de farine de bois sont contenues en une quantité de 0,5 à 30 % en poids de la totalité de l'agent nettoyant pour la peau.

10. Utilisation conformément à la revendication 8 ou à la revendication 9,
**caractérisée en ce que**
comme agent épaississant soluble dans l'eau, on utilise de la gomme xanthane.

11. Utilisation conformément à l'une quelconque des revendications 8 à 10,
**caractérisée en ce que**
l'agent nettoyant pour la peau contient des substances actives sur la tension superficielle anionique, zwitterioniques, ou ampholytiques.

12. Utilisation conformément à la revendication 11,
**caractérisée en ce que**
l'agent nettoyant pour la peau contient des substances actives sur la tension superficielle non ioniques.
